# EUROPÄISCHE PATENTSCHRIFT

(11) **EP 2 699 308 B1**
(45) Veröffentlichungstag und Bekanntmachung des Hinweises auf die Patenterteilung: **07.06.2017**
(21) Anmeldenummer: 12715602.4
(22) Anmeldetag: 11.04.2012
(51) Int. Cl.: A61N 1/04, A61F 13/00

(54) **BANDAGE UND ELEKTRODENSYSTEM**
BANDAGE AND ELECTRODE SYSTEM
BANDAGE ET SYSTÈME D'ÉLECTRODES

(30) Priorität: 18.04.2011 DE 102011018470
(43) Veröffentlichungstag der Anmeldung: 26.02.2014
(73) Patentinhaber: Otto Bock HealthCare GmbH, 37115 Duderstadt (DE)
(72) Erfinder: KROLL-ORYWAHL, Olaf, 37083 Göttingen (DE); REINHARDT, Holger, 47906 Kempen (DE); ALBRECHT-LAATSCH, Erik, 37077 Göttingen (DE)
(74) Vertreter: Friedrich, Andreas
(86) Internationale Anmeldenummer: PCT/EP2012/001566
(87) Internationale Veröffentlichungsnummer: WO 2012/143100

(56) Entgegenhaltungen:
- GB-A- 342 419
- GB-A- 2 400 915
- US-A- 4 445 518
- US-A1- 2003 114 892

## Beschreibung

Die Erfindung betrifft eine Bandage mit wenigstens einem Trägerelement und wenigstens zwei voneinander beabstandeten Elektroden. Die Erfindung betriff zudem ein Elektrodensystem für eine derartige Bandage. Eine derartige Bandage ist beispielsweise aus der DE 10 2004 009 210 A1 bekannt. Das wenigstens eine Trägerelement ist in diesem Fall ein elastischer Gurt, der beispielsweise um den Körper eines Patienten herumgelegt werden kann. Das wenigstens eine Trägerelement kann jedoch auch in anderer Form ausgestaltet sein, wie es beispielsweise für Knieorthesen, Unterschenkelorthesen oder sonstige Bandagensysteme bekannt ist.

Derartige Systeme werden heute oftmals mit Elektroden ausgestattet, um elektrotherapeutische Ströme in den menschlichen Körper eindringen zu lassen und somit das Muskelwachstum und das Muskeltraining zu stimulieren.

Um hier eine optimale Wirkung zu erreichen, ist es wichtig, dass zwischen der Elektrode und der Haut des Trägers der Bandage ein elektrischer Kontakt hergestellt ist. Dabei stellt sich das Problem, dass sich der Träger des Bandagensystems bzw. der Bandage ständig in Bewegung befindet. Gerade in der Rehabilitationsphase nach einer Verletzung oder Krankheit ist eine Aktivierung der Aktivität des Patienten von grundlegender Bedeutung. Soll die Elektrostimulation bzw. Elektrotherapie erfolgreich verlaufen, ist auch unter diesen Bedingungen eine permanente Kontaktierung der Elektroden während der Bewegung der Muskulatur wichtig.

Verrutscht während der Bewegung des Trägers der Bandage die Elektrode so, dass die Kontaktfläche zur menschlichen Haut verringert wird, entstehen bei gleichem in den Körper eingebrachten Strom deutlich höhere Stromdichten aufgrund der nun kleineren Kontaktfläche. Dadurch kann es zu lokalen Verbrennungen kommen. Gleiches gilt, wenn der Träger der Bandage unter der Elektrode zu schwitzen beginnt. Dadurch wird die Leitfähigkeit, also der elektrische Kontakt zwischen der Elektrode und der menschlichen Haut, deutlich erhöht, so dass es bei gleicher Spannung zu einer lokalen Stromerhöhung kommt.

Aus der US 2002/0128686 A1 ist eine Vorrichtung zum Stimulieren der Abdominalmuskulatur eines Patienten bekannt. Dabei werden verschiedene Elektroden an der Haut des Patienten angeordnet. Um eine besonders gute elektrische Kontaktierung zu erreichen, ist die dem Patienten zugewandte Seite der Elektrode mit einer elektrisch leitfähigen Beschichtung, beispielsweise in Form eines Gels, beschichtet. Diese so genannten Gelelektroden, die an der Haut des Patienten kleben, haben den Nachteil, Hautirritationen erzeugen zu können und Falten zu bilden, wodurch wiederum ungewollte elektrische Spannungsspitzen entstehen können, die teilweise zu Verbrennungen führen. Zudem ist es für den Träger unangenehm, über längere Zeit die an dem Körper klebenden Elektroden zu tragen.

Aus der US 2010/0004715 A1 ist eine Vorrichtung für die Elektrotherapie bekannt, die über wenigstens eine Elektrode verfügt, durch die Muskelkontraktionen angeregt werden können. Die dort beschriebene Vorrichtung verfügt zudem über Sensoren, die Signale aufnehmen und an eine Steuereinheit weiterleiten, die von den elektrisch stimulierten Muskelgruppen als Antwort auf die elektrische Stimulation abgegeben werden. Auf diese Weise sollen die Position und die Wirkung der Elektroden optimiert werden und eine Fehlfunktion der Elektroden, die beispielsweise eine Überhitzung sein kann, schnell erkannt werden. Dies ist insbesondere bei Patienten von Vorteil, die komatös, anästhesiert oder auf eine sonstige Weise nicht in der Lage sind, mit ihrer Umwelt zu kommunizieren.

Aus der EP 0 948 972 A2 ist eine Vorrichtung bekannt, mit der ein Defibrilator am Körper des Patienten mittels mehrerer Gurte getragen werden kann. Dabei können die Elektroden in elastischen Taschen angeordnet sein, die beispielsweise aus einem elektrischen nicht leitenden Gewebe bestehen können. Damit wird der direkte Kontakt zwischen der Elektrode und der Haut des Trägers vermieden. Ermitteln nun dafür vorgesehene Sensoren, dass Strom durch die Elektroden in den Körper des Patienten eingeleitet werden muss, wird ein elektrisch leitfähiges Gel verwendet, das aus der Elektrode austritt und einfach durch das Gewebe hindurch treten kann. Dadurch wird die elektrische Kontaktierung in dem Moment gewährleistet. Dieses Verfahren ist jedoch nur möglich, wenn, wie bei einem Defibrilator, nur in Ausnahmefällen und nur über einen sehr kurzen Zeitraum Stromstöße in den Körper des Patienten eingebracht werden müssen. Zur elektrischen Stimulation von Muskeln, die über einen längeren Zeitraum erfolgen muss, ist diese Lösung unbrauchbar.

Um zu gewährleisten, dass selbst für den Fall, dass die Elektroden sich während der Bewegung des Trägers der Bandage relativ zu dessen Haut nicht bewegen, muss sich ein Anpressdruck der Elektroden an die Haut des Trägers der Bandage in einem bestimmten Bereich befindet. Dies wird insbesondere dann schwierig, wenn sich der Träger der Bandage beispielsweise im Sitzen gegen die Rückenlehne eines Stuhls lehnt oder sich im Sitzen vorbeugt, so dass kein Kontakt mehr zur Lehne besteht. In beiden Fällen ändert sich der Anpressdruck der Elektrode an die Haut des Trägers der Bandage, so dass die elektrische Kontaktierung, und insbesondere die elektrische Leitfähigkeit der Verbindung zwischen Elektrode und Haut, deutlich geändert wird.

Die DE 10 2004 009 210 A1 schlägt dafür vor, die Elektroden an einer elastischen Bandage anzubringen. Im Bereich der Elektroden soll an der elastischen Bandage eine Tasche angeordnet werden, die aus einem wesentlich unelastischeren Material als die Bandage besteht. Die Tasche aus dem unelastischen Material enthält ein elastisches Kissen, das beispielsweise in Form eines aufblasbaren Schlauches ausgebildet sein kann. Wird dieser Schlauch nun aufgeblasen, kann sich die Tasche wegen ihrer weitgehenden Unelastizität nur in Richtung auf die Elektrode ausdehnen und drückt diese somit stärker gegen die Haut des Patienten.

Nachteilig ist, dass für die Elektroden eine unelastische Tasche an der ansonsten elastischen Bandage angeordnet werden muss. Damit ist man in der Positionierung der Elektroden nicht mehr flexibel, sondern die Position, an der Elektroden an der Bandage vorgesehen sein können, wird bereits herstellerseitig festgelegt. Zudem muss ein separates Bauteil an der Bandage angeordnet werden, wodurch der Produktions- und Kostenaufwand steigt.

Nachteilig ist zudem, dass zwar durch den Füllgrad des aufblasbaren Schlauches der Druck, mit dem die Elektrode auf die Haut des Patienten gedrückt wird, einstellbar ist, sich dieser jedoch beispielsweise bei Bewegung des Patienten stark ändern kann. Insbesondere ist es möglich, dass der Druck auf einige Elektroden durch eine Bewegung oder ein Anlehnen des Patienten an einen Gegenstand, beispielsweise eine Rückenlehne eines Stuhles, stark ansteigt, während er bei anderen Elektroden deutlich abfällt. Dieser unterschiedlichen Druckentwicklung bei unterschiedlichen Elektroden kann mit der im Stand der Technik vorgeschlagenen Lösung nicht Rechnung getragen werden.

Aus der US 2003/0114892 A1 ist eine neuroprothetische Vorrichtung bekannt, die Elektroden aufweist, um eine Elektrostimulation der Muskeln des Amputationsstumpfes herbeizuführen. Die Vorrichtung verfügt dabei unter eine Schale, an deren Innenseite, die dem Amputationsstumpf zugewandt, Kissen angeordnet sind, die als Träger für die eigentlichen Elektroden dienen. Die Kissen dienen dazu, Druck auf die Elektroden und damit auf das unterliegende Gewebematerial des Amputationsstumpfes zu übertragen und weisen eine Elastizität auf, die in etwa der menschlichen Gewebes entspricht.

Aus der US 4,445,518 ist ein Verfahren bekannt, eine Elektrode an der Innenseite eines Gipsverbandes anzuordnen. Dabei weist die Elektrode eine leitfähige Oberfläche für den Kontakt der Hautoberfläche sowie eine gegenüberliegende Haltefläche auf, mit der sie an der Innenseite des Gipsverbands angeordnet wird.

Die GB 2 400 915 A offenbart einen Elektrodengürtel für eine Impedanztomografie, der wenigstens 16 Elektroden auf einem elastischen Träger umfasst, der um den Körper des zu untersuchenden Patienten gelegt wird.

Aus der GB 342 419 A sind Elektroden für therapeutische Anwendungen bekannt, die an einem Kissen angeordnet sind, das mit einem Feststoff, beispielsweise Metallwolle, Lametta oder Brokat gefüllt ist.

Der in Anspruch 1 definierten Erfindung liegt somit die Aufgabe zugrunde, eine gattungsgemäße Bandage so zu verbessern, dass die Positionierung der Elektroden am wenigstens einen Trägerelement flexibel erfolgen kann und der unter-schiedlichen Druckentwicklung bei verschiedenen Elektroden einfach Rechnung getragen werden kann.

Die Erfindung löst die gestellte Aufgabe durch eine gattungsgemäße Bandage, die sich dadurch auszeichnet, dass an dem wenigstens einen Trägerelement wenigstens zwei mit einem Fluid füllbare Kissen angeordnet sind, und an jedem Kissen wenigstens eine Elektrode befestigt ist, wobei die wenigstens zwei Kissen derart über wenigstens eine Fluidverbindung miteinander verbunden sind, dass ein Innendruckausgleich zwischen zumindest zwei der Kissen stattfinden kann.

An dem wenigstens einen Trägerelement der Bandage, das beispielsweise ein Gurt sein kann, befinden sich folglich wenigstens zwei mit einem Fluid befüllbare, beispielsweise aufblasbare, Kissen. An einer dem Trägerelement abgewandten Seite eines jeden Kissens befindet sich wenigstens eine Elektrode. Zwischen den wenigstens zwei voneinander beabstandeten Elektroden und dem wenigstens einen Trägerelement befinden sich folglich unterschiedliche Kissen. Diese sind über wenigstens eine Fluidverbindung so miteinander verbunden, dass ein Druckausgleich der Innendrücke der beiden Kissen stattfinden kann. Auf diese Weise ist gewährleistet, dass in jedem der Kissen in jedem Moment der gleiche Druck herrscht, da es zum Druckausgleich zwischen den Druckkissen kommt. Dadurch herrscht der gleiche Druck natürlich auch in der Fluidverbindung. Selbst wenn folglich eines oder mehrere der Kissen durch eine Bewegung oder Anlehnen des Patienten stärker belastet werden, wird der Druck nicht nur lokal auf diese Elektrode ausgeübt, sondern über den Druckausgleich durch die wenigstens eine Fluidverbindung auch auf alle anderen Kissen übertragen. Eine besondere Belastung einer oder mehrerer der Elektroden bzw. der sich an ihnen befindlichen Kissen geht zumeist mit einer Entlastung anderer Elektroden einher, die an dem wenigstens einen Trägerelement angeordnet sind. So ist es beispielsweise bei einer Rückenbandage bei einem Anlehnen an die Rückenlehne eines Stuhles regelmäßig so, dass die Elektroden, die sich im Rückenbereich des Patienten befinden, starkem Druck ausgesetzt sind, während die Elektroden, die im Bauchbereich des Patienten angeordnet sind, deutlich entlastet werden. Durch die Verbindung der verschiedenen Kissen über die wenigstens eine Fluidverbindung ist hier ein Druckausgleich möglich, so dass weiterhin alle Elektroden gleich belastet und an die Haut des Patienten gedrückt werden.

Durch die erfindungsgemäße Ausgestaltung einer Bandage ist es zudem möglich, die aus dem Stand der Technik bekannten Taschen aus einem im Wesentlichen unelastischen Material einzusparen. Dadurch wird nicht nur der Konstruktions- und Kostenaufwand reduziert, sondern gleichzeitig die Flexibilität bei der Positionierung der Elektroden deutlich erhöht. So ist es möglich, die Elektroden mit einem bereits daran befestigten Kissen beispielsweise über Klettverschlüsse, Druckknöpfe oder ähnliches frei am wenigstens einen Trägerelement der Bandage zu befestigen. Dadurch ist es möglich, die gleiche Bandage für die Stimulation unterschiedlicher Muskelgruppen zu verwenden. Zudem wird auch die Elastizität der Bandage im Bereich der Elektroden nicht eingeschränkt.

Die Elektroden können dabei nicht nur ausgebildet sein, um elektrische Impulse in den Körper zu übertragen. Es können auch EMG-Elektroden sein (EMG = Elektromyogramm), durch die Signale vom Körper aufgenommen werden können. Auch eine Kombination verschiedener Elektroden an einer Bandage und in einem Elektrodensystem ist denkbar.

In einer besonders vorteilhaften Ausgestaltung ist an jedem Kissen genau eine Elektrode angeordnet. Alternativ dazu können auch größere Kissen vorgesehen sein, an denen mehr als eine Elektrode angeordnet ist. Dies ist insbesondere dann sinnvoll, wenn mehrere Elektroden sehr nah bei einander am Körper des Trägers der Bandage angeordnet werden sollen. Sobald die Elektroden jedoch voneinander beabstandet sind, bietet es sich an, für jede Elektrode ein separates Kissen vorzusehen. Dadurch kann insbesondere die Menge des Fluids, die in die Kissen eingefüllt ist, deutlich reduziert werden, was insbesondere dann von Vorteil ist, wenn die Kissen nicht mit Luft, sondern beispielsweise mit einer Spezialflüssigkeit befüllt sind. Vorzugsweise umfasst die Bandage eine Pumpe, die eingerichtet ist, das Fluid in die Kissen hinein und/oder aus den Kissen hinaus zu pumpen. Damit kann der Füllgrad der Kissen individuell auf den Anwendungsfall angepasst werden. Die Pumpe ist vorzugsweise elektrisch betreibbar. Damit ist eine besonders einfache Steuerung des Füllgrades möglich. Sind die Elektroden beispielsweise für Stellen vorgesehen, an denen normalerweise ein gewisser Abstand zwischen der Haut des Patienten und der angelegten Bandage vorliegt, können die Kissen stärker befüllt werden, so dass sie eine größere Ausdehnung erreichen und die Elektroden über den vorhandenen Abstand hinweg so an die Haut des Patienten andrücken, dass eine optimale Kontaktierung gewährleistet ist. Sind die Elektroden hingegen für Stellen vorgesehen, die direkt an einer angelegten Bandage anliegen, ist ein weniger starker Füllgrad ausreichend, so dass das Fluid, das sich noch in den Kissen befindet, zum Teil abgelassen bzw. herausgepumpt werden kann.

In einer bevorzugten Ausgestaltung ist wenigstens ein Sensor zum Messen des Innendrucks vorgesehen. Ein solcher Sensor kann beispielsweise in einem der Kissen, in der wenigstens eine Fluidverbindung oder in der Pumpe angeordnet sein. Jede Positionierung, die eine Bestimmung des Innendruckes, der in den Kissen herrscht, erlaubt, möglich. Vorzugsweise umfasst die Bandage dann auch eine elektrische Steuerung, die eingerichtet ist, die Pumpe in Abhängigkeit von den gemessenen Werten des Innendrucks zu steuern. Damit ist es möglich, auch während des Tragens der Bandage beispielsweise über einen längeren Zeitraum, den Innendruck des Fluids in den Kissen anzupassen und nachzuregeln. Somit ist gewährleistet, dass immer der gewünschte Druck herrscht. Lehnt sich beispielsweise der Träger einer Bandage an die Rückenlehne eines Stuhles an, wird in diesem Bereich der Druck auf die Elektrode stark erhöht. Dadurch wird insgesamt der Druck im System aus den Kissen und der wenigstens einen Fluidverbindung erhöht, so dass es von Vorteil sein kann, einen Teil des Fluids aus den Kissen bzw. der Fluidverbindung herauszupumpen. Dadurch wird der Druck wieder reduziert, so dass der gewünschte voreingestellte Solldruck erreicht werden kann. Alternativ kann es auch von Vorteil sein, während des Tragens der Bandage einen zusätzlichen Anteil Fluid in das System aus Kissen und wenigstens einer Fluidverbindung hineinzupumpen, um den Druck wieder zu erhöhen, etwa wenn die Bandage und/oder die Elektroden derart verrutschen, dass der Druck in den Kissen und damit auch der Anpressdruck der Elektrode an die Haut des Trägers der Bandage abnimmt. Dies ist durch die elektrische Steuerung einfach möglich, so dass immer der optimale Druck eingeregelt werden kann.

Als vorteilhaft hat sich herausgestellt, wenn zwischen wenigstens einem Kissen und dem wenigstens einen Trägerelement ein Plattenelement vorgesehen ist, das eine geringere Elastizität aufweist als das Trägerelement. Auf diese Weise wird insbesondere bei sehr elastischen Trägerelementen, beispielsweise elastischen Bandagen oder Gurten, erreicht, dass ein Aufpumpen der Kissen mit dem Fluid nicht dazu führen kann, dass sich das Kissen in die von der Elektrode weggerichtete Richtung ausdehnen kann. Durch das Plattenelement mit seiner geringen Elastizität ist das Kissen gezwungen, sich in Richtung auf die Elektrode auszudehnen, so dass die Elektrode stärker in Richtung auf den Patienten gedrückt wird. Das vorgesehene Plattenelement erfüllt damit die Aufgabe der im Wesentlichen unelastischen Tasche aus dem Stand der Technik. Vorteilhaft ist hier jedoch, dass das Plattenelement hier zwischen dem Kissen und dem wenigstens einen Trägerelement vorgesehen ist, so dass es die Flexibilität bei der Wahl der Position Elektroden am wenigstens einen Trägerelement nicht beeinträchtigt.

Alternativ dazu ist es auch möglich, kein Plattenelement zu verwenden, so dass sich die Kissen in beide Richtungen frei wölben können.

Jedes der Kissen kann aus mehreren Folienelementen zusammengesetzt sein. Diese können beispielsweise mittels Folienultraschallschweißen, die beispielsweise aus der Fertigung von Luftmatratzen bekannt ist, oder mittels einer Folienklebetechnik miteinander verbunden werden. Alternativ können natürlich auch Reibschweißverfahren, Laserverfahren oder Hochfrequenzschweißverfahren verwendet werden. Vorzugsweise können die Elektroden an einer dem Kissen abgewandten Seite eine Klebeschicht zum Ankleben an die Haut eines Patienten aufweisen. Damit kann die Kontaktierung weiter verbessert werden.

Ein erfindungsgemäßes Elektrodensystem verfügt über wenigstens zwei Elektroden und wenigstens zwei Kissen, wobei an jedem Kissen wenigstens eine Elektrode angeordnet ist. Zudem sind die Kissen über wenigstens eine Fluidverbindung derart miteinander verbunden, dass ein Innendruckausgleich zwischen zumindest zwei der Kissen stattfinden kann. An vorzugsweise wenigstens einem Kissen ist an einer der an ihm angeordneten Elektrode abgewandten Seite ein Plattenelement vorgesehen.

Vorzugsweise ist sowohl bei einem Elektrodensystem als auch bei einer Bandage zudem an jedem Kissen an der den Elektroden abgewandten Seite ein Befestigungsmittel, beispielsweise ein Klettverschluss vorgesehen, mit dem das Elektrodensystem an einem Gurt oder einer Bandage befestigt werden kann. Auf diese Weise kann eine herkömmliche Bandage, an der herstellerseitig keine Elektroden vorgesehen sind, mit Elektroden nachgerüstet werden. Durch die flexible Anbringung der einzelnen Kissen und der daran angeordneten Elektroden kann diese für die Stimulation verschiedenster Muskelgruppen verwendet werden. Insbesondere kann das Elektrodensystem nach dem Ende der Stimulationstherapie von dem Gurt oder der Bandage entfernt und mit einer anderen Bandage für andere Muskelgruppen benutzt werden. Damit wird das Elektrodensystem recyclebar, so dass nicht für jede Erkrankung oder gegebenenfalls sogar nicht für jeden Patienten, beispielsweise im Krankenhaus oder in einer Rehabilitationsklinik, eine neue individuell angefertigte Bandage hergestellt werden muss. Dadurch sinken die Behandlungskosten deutlich.

Über eine insbesondere elektrische Steuerung werden sowohl die Elektroden gesteuert, über die elektrische Impulse an den Körper abgegeben werden sollen, als auch die von EMG-Elektroden aufgenommenen elektrischen Signale weiterverarbeitet. In einer bevorzugten Ausführungsform ist die elektrische Steuerung in einem die Pumpe umgebenden Gehäuse angeordnet. Damit ist es für den Träger der Bandage möglich, an dieser Stelle sowohl Einstellungen an der Pumpe und damit am Fülldruck der Kissen vorzunehmen als auch beispielsweise die Abfolge der abgegebenen elektrischen Impulse zu steuern. Es muss in dieser Ausgestaltung nur ein Bauteil, nämlich das kombinierte Gehäuse für die Pumpe und die Steuerung, außen an der Bandage angeordnet werden.

Mit Hilfe einer Zeichnung wird nachfolgend ein Ausführungsbeispiel der vorliegenden Erfindung näher erläutert. Es zeigt
- Figur 1 -: eine schematische Draufsicht auf eine Bandage gemäß einem ersten Ausführungsbeispiel der vorliegenden Erfindung,
- Figur 2 -: die schematische Darstellung eines Elektrodensystems gemäß einem Ausführungsbeispiel der vorliegenden Erfindung,
- Figur 3a -: einen schematischen Querschnitt durch ein Trägerelement im Bereich eines Kissens,
- Figur 3b -: die Darstellung aus Figur 3a mit einer daran angeordneten Elektrode und
- Figur 4 -: einen Wirkungsplatz für die Regelung des Innendrucks in den Kissen.

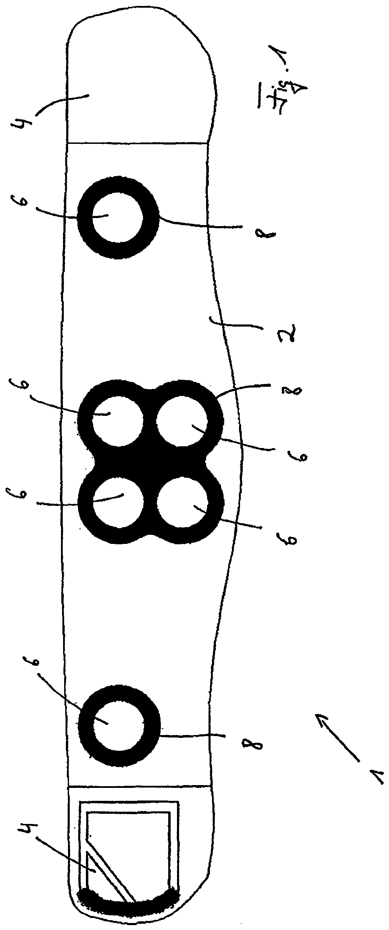

Figur 1 zeigt die schematische Draufsicht auf eine Bandage 1 gemäß einem ersten Ausführungsbeispiel der vorliegenden Erfindung. Diese Bandage 1 weist ein Trägerelement 2 auf, das im in Figur 1 gezeigten Ausführungsbeispiel in Form eines Gurtes vorliegt. An den rechten und linken Enden des Trägerelementes 2 befinden sich Schließelemente 4, die beispielsweise als Zweiteiler eines Klettverschlusses ausgebildet sein können. Alternativ sind hier natürlich auch Schnallen, Druckknöpfe und ähnliche Ausgestaltungen denkbar.

Die in Figur 1 gezeigte Bandage 1 verfügt über sechs Elektroden 6, die jeweils auf einem Kissen 8 angeordnet sind. Die Kissen 8 sind am Trägerelement 2 angeordnet, das hier die Form eines Gurtes aufweist. Es sind auch andere Ausgestaltungen des Trägerelementes 2 denkbar. Für die Erfindung wichtig ist lediglich, dass die Bandage 1 über wenigstens ein Trägerelement 2 verfügt, an dem die Kissen 8 angeordnet sind.

Die in Figur 1 gezeigte Ansicht zeigt folglich die dem Körper des Patienten zugewandte Seite der Bandage 1. Die jeweils rechte und linke Elektrode 6 ist auf einem separaten Kissen 8 angeordnet, währen die vier im mittleren Bereich des Trägerelementes 2 angeordneten Elektroden 6 auf einem gemeinsamen Kissen 8 ruhen. Die Kissen 8 sind untereinander durch nicht gezeigte Fluidverbindungen verbunden, so dass immer ein Druckausgleich des Innendrucks der verschiedenen Kissen 8 stattfinden kann. Eine solche Fluidverbindung kann beispielsweise durch einen Schlauch 10 oder einen Kanal gebildet sein. Es kann auch eine luftdicht verschweißte Folie zur Verbindung der Kissen verwendet werden.

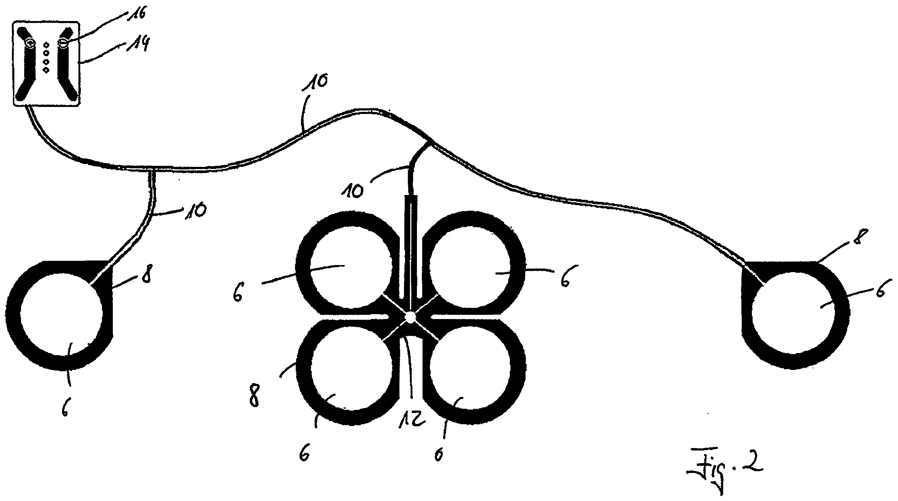

Figur 2 zeigt eine schematische Darstellung eines Elektrodensystems mit Elektroden 6 und den darunter liegenden Kissen 8. Jedes der Kissen 8 verfügt über einen Hohlraum, der mit einem Fluid befüllbar ist. Diese Hohlräume sind in Figur 2 deckungsgleich mit den Elektroden 6. Über mehrere Schläuche 10, die im gezeigten Ausführungsbeispiel die Fluidverbindung bilden, sind die verschiedenen Kissen 8 miteinander verbunden. Das gemeinsame Kissen 8 für die vier im mittleren Bereich der in Figur 1 gezeigten Bandage 1 angeordneten Elektroden 6 verfügt über vier mit dem Fluid befüllbare Kammern. Diese sind innerhalb des Kissens 8 über eine kreuzförmige Verbindung 12 miteinander verbunden, so dass auch hier ein Innendruckausgleich stattfinden kann.

An den Verbindungsschlauch 10 angeschlossen ist eine schematisch dargestellte Pumpe 14. Diese umfasst zudem eine elektrische Steuerung 16, die eingerichtet ist, die Pumpe 14 zu steuern. Durch diese Pumpe ist es möglich, sowohl ein Medium durch die Schläuche 10 in die Kissen 8 hineinzupumpen als auch Medium aus den Kissen 8 abzulassen. Damit kann der Innendruck und damit auch der Anpressdruck der Elektroden an den Körper des Trägers individuell eingestellt werden.

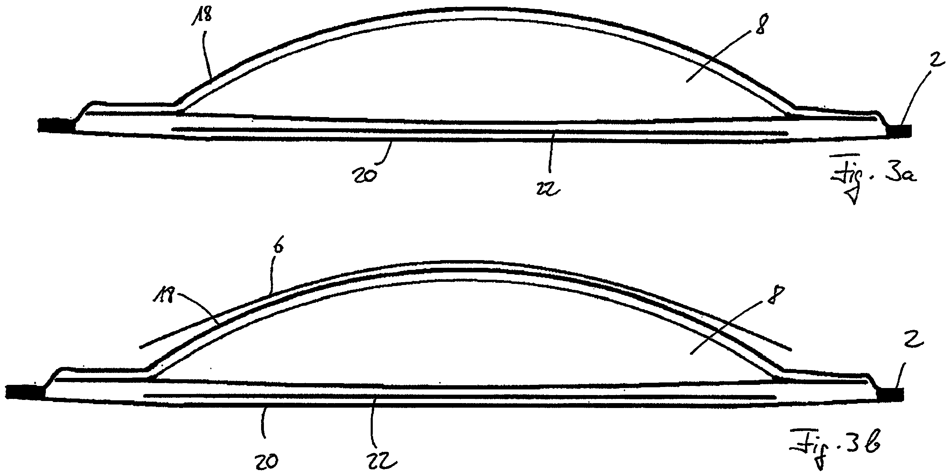

Figur 3a zeigt einen Durchschnitt durch ein Trägerelement 2 im Bereich eines Kissens 8. Im in den Figuren 3a und 3b gezeigten Ausführungsbeispiel ist das Kissen 8 in das Trägerelement 2 integriert. Man erkennt, dass zwischen einer dem Patienten zugewandten inneren Wandung 18 und einer auf der entgegengesetzten Seite liegenden äußeren Wandung 20 angeordnet ist. Auf diese Weise ist sichergestellt, dass das Kissen 8 relativ zum Trägerelement 2 nicht verrutschen kann. Alternativ dazu kann das Kissen 8 auch durch Befestigungselemente wie beispielsweise einen Klettverschluss oder ähnliche auf der dem Patienten zugewandten Seite des Trägerelementes angeordnet werden. In diesem Fall ist die Flexibilität bei der Positionierung der Kissen 8 und damit auch der auf den Kissen 8 anzuordnenden Elektroden 6 deutlich erhöht. Es besteht jedoch die Gefahr, dass sich das Kissen 8 und damit auch die Elektrode 6 vom Trägerelement 2 lösen und relativ zu diesem verschoben werden können.

Zwischen dem Kissen 8 und der äußeren Wandung 20 des Trägerelementes 2 ist schematisch ein Plattenelement 22 dargestellt. Wie in den Figuren 3a und 3b gezeigt, verhindert dieses Plattenelement 22 wirksam, dass sich das Kissen 8 auch in Richtung auf die äußere Wandung 20 des Trägerelementes 2 ausdehnt, wenn es mit dem Fluid, beispielsweise Luft, gefüllt wird.

Figur 3b zeigt die Situation aus Figur 3a mit einer an der inneren Wandung 18 des Trägerelementes 2 angeordneten Elektrode 6. Man erkennt, dass ein Aufblasen des Kissens 8 bzw. ein Einpumpen des Fluides in das Kissen 8 eine Ausdehnung des Kissens 8 nur in Richtung auf die innere Wandung 18 des Trägerrelementes 2 zur Folge hat. Die an dieser Seite angeordnete Elektrode 6 wird somit in Richtung auf den Patienten bzw. den Träger der Bandage 1 gedrückt, so dass es zu einer optimalen Kontaktierung der Haut des Trägers kommt. Auf diese Weise ist sichergestellt, dass die Kontaktfläche zeitlich konstant bleibt und nicht durch ein Verrutschen, Verschieben oder Ablösen der Elektroden erhöht oder reduziert wird. Damit ist sicher, dass die optimalen Stromimpulse und Signale in den Körper des Trägers der Bandage 1 eindringen und so die Muskelstimulation auf bestmögliche Weise erfolgen kann.

Im in Figur 3b gezeigten Ausführungsbeispiel ist die Elektrode 6 beispielsweise über Klebestellen, Klettverschlüsse oder ähnliche Befestigungsvorrichtungen an der inneren Wandung 18 des Trägerelementes 2 befestigt. Alternativ dazu kann die Elektrode 6 auch direkt am Kissen 8 befestigt sein, wenn diese an der dem Träger der Bandage 1 zugewandten Seite der inneren Wandung 18 des Trägerelementes 2 angeordnet ist. Auf diese Weise lässt sich die Kombination aus Kissen 8 und Elektrode 6 leicht relativ zum Trägerelement 2 versetzen, so dass die gleiche Bandage 1 verwendet werden kann, um unterschiedliche Muskelgruppen an unterschiedlichen Stellen des menschlichen Körpers zu stimulieren.

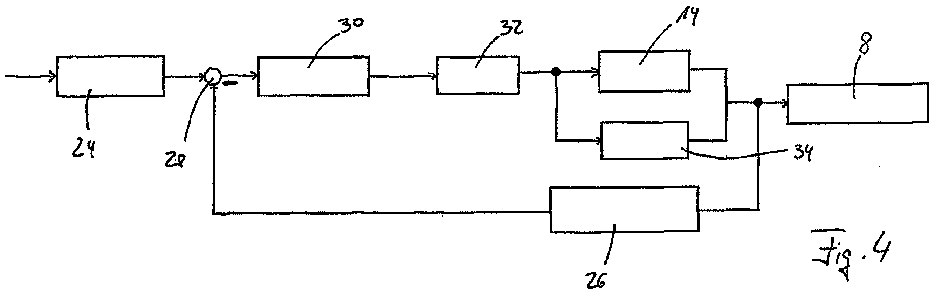

Figur 4 zeigt schematisch einen Wirkungsplan für die Steuerung des Innendrucks in den Luftkammern. Von links kommend wird eine Sollgröße 24 vorgegeben, die dem gewünschten Innendruck in den Luftkammern der Kissen 8 entspricht. Am ganz rechten Ende des Wirkungsplans befinden sich die Kissen 8. Durch einen in den Figuren bisher nicht gezeigten Sensor 26, der beispielsweise direkt in einem der Kissen 8 oder in der Pumpe 14 angeordnet sein kann, wird unmittelbar vor den Kissen 8 der Innendruck gemessen. Dieser wird in einem Vergleicher 28 mit der Sollgröße 24, also dem gewünschten Innendruck verglichen. Sofern sich hier Abweichungen ergeben, wird über ein Regelglied 30 und einen Steller 32 ein Stellglied angesteuert, um den durch den Sensor 26 gemessenen Innendruck in den Kissen 8 zu verändern. Dies kann einerseits die in Figur schematisch dargestellte Pumpe 14 oder ein Ventil 34 sein. Ist der durch den Sensor 26 gemessene Innendruck in den Kissen 8 zu groß im Vergleich zu den als Sollgröße 24 vorgesehenen gewünschten Innendruck, wird über das Ventil 34 ein Teil des sich in den Kissen 8 befindenden Fluids abgelassen. Dadurch sinkt der Innendruck und nähert sich der gewünschten Sollgröße 24 an. Ist der durch den Sensor 26 gemessene Innendruck jedoch größer als der als Sollgröße 24 vorgegebene Innendruck, wird über die Pumpe 14 der Innendruck in den Kissen 8 erhöht, indem Fluid in die Kissen 8 eingepumpt wird.

### Bezugszeichenliste

- 1: Bandage
- 2: Trägerelement
- 4: Schließelement
- 6: Elektrode
- 8: Kissen
- 10: Schlauch
- 12: Verbindung
- 14: Pumpe
- 16: elektrische Steuerung
- 18: innere Wandung
- 20: äußere Wandung
- 22: Plattenelement
- 24: Sollgröße
- 26: Sensor
- 28: Vergleicher
- 30: Regelglied
- 32: Steller
- 34: Ventil

## Patentansprüche

1. Elektrodensystem für eine Bandage (1), die ein Trägerelement (2) aufweist, wobei das Elektrodensystem wenigstens zwei Elektroden (6) und wenigstens zwei mit einem Fluid füllbaren Kissen (8), die an dem Trägerelement angeordnet werden können, aufweist, wobei an jedem Kissen (8) wenigstens eine Elektrode (6) angeordnet ist und wobei die Kissen (8) über wenigstens eine Fluidverbindung derart miteinander verbunden sind, dass ein Innendruckausgleich zwischen den wenigstens zwei Kissen (8) stattfinden kann.

2. Elektrodensystem nach Anspruch 1, **dadurch gekennzeichnet, dass** an wenigstens einem Kissen (8) an einer der an ihm angeordneten Elektrode (6) abgewandten Seite ein Plattenelement (22) vorgesehen ist.

3. Bandage (1) mit wenigstens einem Trägerelement (2) und einem Elektrodensystem nach Anspruch 1 oder 2, **dadurch gekennzeichnet, dass** an dem wenigstens einen Trägerelement (2) die wenigstens zwei mit einem Fluid füllbare Kissen (8) angeordnet sind.

4. Bandage (1) nach Anspruch 3, **dadurch gekennzeichnet, dass** an jedem Kissen (8) genau eine Elektrode (6) angeordnet ist.

5. Bandage (1) nach Anspruch 3 oder 4, **dadurch gekennzeichnet, dass** sie eine Pumpe (14) umfasst, die eingerichtet ist, das Fluid in die Kissen (8) hinein und/oder aus den Kissen (8) hinaus zu pumpen.

6. Bandage (1) nach Anspruch 5, **dadurch gekennzeichnet, dass** die Pumpe (14) elektrisch betreibbar ist.

7. Bandage (1) nach einem der Ansprüche 3 bis 6, **dadurch gekennzeichnet, dass** wenigstens ein Sensor (26) zum Messen eines Innendrucks vorgesehen ist.

8. Bandage (1) nach Anspruch 7, **dadurch gekennzeichnet, dass** sie eine elektrische Steuerung (16) umfasst, die eingerichtet ist, die Pumpe (14) in Abhängigkeit von den gemessenen Werten des Innendruckes zu steuern.

9. Bandage (1) nach einem der Ansprüche 3 bis 8, **dadurch gekennzeichnet, dass** zwischen wenigstens einem Kissen (8) und dem wenigstens einen Trägerelement (2) ein Plattenelement (22) vorgesehen ist, das eine geringere Elastizität aufweist als das Trägerelement (2).

10. Bandage (1) nach einem der Ansprüche 3 bis 9, **dadurch gekennzeichnet, dass** jedes der Kissen (8) aus mehreren Folienelementen zusammengesetzt ist.

11. Bandage (1) nach einem der Ansprüche 5 bis 10, **dadurch gekennzeichnet, dass** in einem die Pumpe (14) umgebenden Gehäuse eine Steuerungsvorrichtung vorgesehen ist, die eingerichtet ist, die Elektroden (6) derart anzusteuern, dass vorbestimmte elektrische Signale abgegeben werden oder von Elektroden (6) aufgenommene Signale weiterverarbeitbar sind.

12. Bandage (1) nach einem der Ansprüche 3 bis 11, **dadurch gekennzeichnet, dass** an den Kissen (8) ein Befestigungselement, beispielsweise ein Element eines Klettverschlusses, angeordnet ist, durch das die Kissen (8) frei an dem Trägerelement (2) positionierbar sind.

## Claims

1. An electrode system for a bandage (1) having a carrier element (2), the electrode system having at least two electrodes (6), and having at least two fluid-fillable pads (8), that can be arranged on the carrier element, wherein at least one electrode (6) is arranged on each pad (8), and wherein the pads (8) are connected to one another via at least one fluid connection so as to allow internal-pressure equalization between the at least two pads (8).

2. The electrode system as claimed in claim 1, **characterized in that** a plate element (22) is provided on at least one pad (8), on a side which is directed away from the electrode (6) arranged thereon.

3. A bandage (1) having at least one carrier element (2) an electrode system according to claim 1 or 2, **characterized in that** the at least two fluid-fillable pads (8) are arranged on the at least one carrier element (2).

4. The bandage (1) as claimed in claim 3, **characterized in that** precisely one electrode (6) is arranged on each pad (8).

5. The bandage (1) as claimed in claim 3 or 4, **characterized in that** it comprises a pump (14), which is intended to pump the fluid into the pads (8) and/or out of the pads (8).

6. The bandage (1) as claimed in claim 5, **characterized in that** the pump (14) can be operated electrically.

7. The bandage (1) as claimed in one of the claims 3 to 6, **characterized in that** there is at least one sensor (26) provided for measuring an internal pressure.

8. The bandage (1) as claimed in claim 7, **characterized in that** it comprises an electrical control means (16), which is intended to control the pump (14) in dependence on the values measured for the internal pressure.

9. The bandage (1) as claimed in one of the claims 3 to 8, **characterized in that** a plate element (22) is provided between at least one pad (8) and the at least one carrier element (2), said plate element having a lower level of elasticity than the carrier element (2).

10. The bandage (1) as claimed in one of the claims 3 to 9, **characterized in that** each of the pads (8) is made up of a plurality of sheet-material elements.

11. The bandage (1) as claimed in one of the claims 5 to 10, **characterized in that** a housing which encloses the pump (14) contains a control device, which is intended to activate the electrodes (6) such that predetermined electrical signals are emitted or signals picked up by electrodes (6) can be subjected to further processing.

12. The bandage (1) as claimed in one of the claims 3 to 11, **characterized in that** a fastening element, for example an element of a touch-and-close fastener, is arranged on the pads (8), and allows the pads (8) to be positioned freely on the carrier element (2).

## Revendications

1. Système d'électrodes pour un bandage (1) qui comprend un élément porteur (2), le système d'électrodes comprenant au moins deux électrodes (6) et au moins deux coussins (8) susceptibles d'être remplis d'un fluide et d'être agencés sur l'élément porteur, au moins une électrode (6) étant agencée sur chaque coussin (8) et les coussins (8) étant reliés entre eux par au moins une liaison fluidique de telle sorte qu'une compensation de pression intérieure peut s'effectuer entre lesdits au moins deux coussins (8).

2. Système d'électrodes selon la revendication 1, **caractérisé en ce qu'**il est prévu sur au moins un coussin (8) un élément formant plaque (22) sur un côté détourné de l'électrode (6) agencée sur celui-ci.

3. Bandage (1) pourvu d'au moins un élément porteur (2) et d'un système d'électrodes selon la revendication 1 ou 2, **caractérisé en ce que** lesdits au moins deux coussins (8) remplissables d'un fluide sont agencés sur ledit au moins un élément porteur (2).

4. Bandage (1) selon la revendication 3, **caractérisé en ce que** précisément une électrode (6) est agencée sur chaque coussin (8).

5. Bandage (1) selon la revendication 3 ou 4, **caractérisé en ce qu'**il comprend une pompe (14) qui est conçue pour pomper le fluide dans les coussins (8) et/ou hors des coussins (8).

6. Bandage (1) selon la revendication 5, **caractérisé en ce que** la pompe (14) fonctionne par voie électrique.

7. Bandage (1) selon l'une des revendications 3 à 6, **caractérisé en ce qu'**il est prévu au moins un capteur (26) pour mesurer une pression intérieure.

8. Bandage (1) selon la revendication 7, **caractérisé en ce qu'**il comprend une commande électrique (16) qui est conçue pour commander la pompe (14) en fonction des valeurs mesurées de la pression intérieure.

9. Bandage (1) selon l'une des revendications 3 à 8, **caractérisé en ce qu'**il est prévu un élément formant plaque (22) entre au moins un coussin (8) et ledit au moins un élément porteur (2), qui présente une élasticité inférieure à celle de l'élément porteur (2).

10. Bandage (1) selon l'une des revendications 3 à 9, **caractérisé en ce que** chacun des coussins (8) est composé de plusieurs éléments en feuille.

11. Bandage (1) selon l'une des revendications 5 à 10, **caractérisé en ce qu'**il est prévu un dispositif de commande dans un boîtier entourant la pompe (14), qui est conçu pour piloter les électrodes (6) de telle sorte que des signaux électriques prédéterminés sont émis ou que des signaux reçus par les électrodes (6) peuvent continuer d'être traités.

12. Bandage (1) selon l'une des revendications 3 à 11, **caractérisé en ce qu'**un élément de fixation, par exemple un élément d'une fermeture agrippante, est agencé sur les coussins (8), qui permet de positionner les coussins (8) librement sur l'élément porteur (2).
